# EUROPÄISCHE PATENTSCHRIFT

(11) **EP 1 448 201 B1**
(45) Veröffentlichungstag und Bekanntmachung des Hinweises auf die Patenterteilung: **28.11.2007**
(21) Anmeldenummer: 02781293.2
(22) Anmeldetag: 28.10.2002
(51) Int. Cl.: A61K 31/495, A61P 3/04, A61P 25/00, A61P 25/18, A61P 25/24, A61P 25/28

(54) **N-(INDOLCARBONYL-)PIPERAZINDERIVATEN ZUR BEHANDLUNG VON FETTSUCHT**
N-(INDOLCARBONYL-)PIPERAZINE DERIVATIVES FOR THE TREATMENT OF OBESITY
DERIVES DE N-(INDOLCARBONYL-)PIPERAZINE DESTINES AU TRAITEMENT DE L'OBESITE

(30) Priorität: 24.11.2001 DE 10157673
(43) Veröffentlichungstag der Anmeldung: 25.08.2004
(73) Patentinhaber: Merck Patent GmbH, 64293 Darmstadt (DE)
(72) Erfinder: VAN AMSTERDAM, Christoph, 64295 Darmstadt (DE); SEDMAN, Ewen, Alresford, Hampshire SO24 0BH (GB); BARTOSZYK, Gard, 64331 Weiterstadt (DE); HELLMANN, Jürgen, 64372 Ober-Ramstadt (DE); VON LANDENGERB, 64291 Darmstadt (DE)
(86) Internationale Anmeldenummer: PCT/EP2002/012009
(87) Internationale Veröffentlichungsnummer: WO 2003/045392

(56) Entgegenhaltungen:
- WO-A-01/07435
- WO-A-99/11641
- US-A- 4 737 501

## Beschreibung

Die Erfindung betrifft die Verwendung von Verbindungen der Formel I 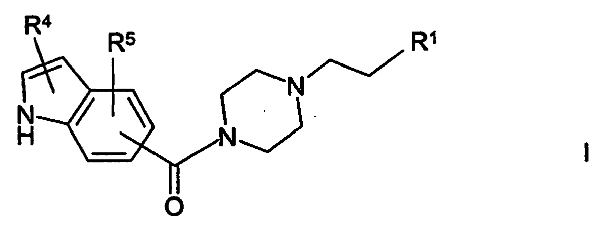 worin
- R¹: einen unsubstituierten oder durch R² und/oder R³ substituierten Phenylrest oder Naphtylrest,
oder Het¹,
- R², R³: jeweils unabhängig voneinander Hal, A, OA, OH oder CN,
- R⁴, R⁵: jeweils unabhängig voneinander H, CN, Acyl, Hal, A, OA, OH, CONH₂, CONHA oder CONA₂,
- R⁴ und R⁵: zusammen auch Alkylen mit 3-5 C-Atomen,
- Het¹: ein- oder zweikerniges unsubstituiertes oder ein- oder zweifach durch Hal, A, OA oder OH substituiertes ungesättigtes hetero cyclisches Ringsystem, welches eines, zwei oder drei gleiche oder verschiedene Heteroatome wie Stickstoff, Sauerstoff und Schwefel enthält,
- A: Alkyl mit 1-6 C-Atomen,
- Hal: F, Cl, Br oder I,
bedeuten,
sowie deren physiologisch unbedenklichen Salzen und Solvaten, zur Herstellung eines Arzneimittels zur Behandlung von Fettsucht.

Für alle Reste, die mehrfach auftreten, wie z.B. A oder Hal, gilt, daß deren Bedeutungen unabhängig voneinander sind.

Der Rest A bedeutet Alkyl und hat 1 bis 6, vorzugsweise 1, 2, 3 oder 4, insbesondere 1 oder 2 C-Atome. Alkyl bedeutet daher insbesondere z.B. Methyl, weiterhin Ethyl, n-Propyl, Isopropyl, n-Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2-oder 1,2,2-Trimethylpropyl.

Acyl hat vorzugsweise 1-6 C-Atome und bedeutet z.B. Formyl, Acetyl, Propionyl, Butyryl, ferner Trifluoracetyl.

Alkylen ist Propylen, Butylen oder Pentylen.

OA ist vorzugsweise Methoxy, ferner auch Ethoxy, n-Propoxy, Isopropoxy, n-Butoxy, Isobutoxy, sek.-Butoxy oder tert.-Butoxy.

Hal bedeutet Fluor, Chlor, Brom oder Iod, insbesondere Fluor oder Chlor.

R¹ ist unsubstituiertes, vorzugsweise - wie angegeben - monosubstituiertes Phenyl oder Naphtyl, im einzelnen bevorzugt Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Trifluormethylphenyl, o-, m- oder p-Hydroxyphenyl, o-, m- oder p-Nitrophenyl, o-, m- oder p-(Trifluormethoxy)-phenyl, o-, m- oder p-Cyanphenyl, o-, m- oder p-Methoxyphenyl, o-, m- oder p-Ethoxyphenyl, o-, m- oder p-Fluorphenyl, o-, m- oder p-Bromphenyl, o-, m- oder p- Chlorphenyl, o-, m- oder p-(Difluormethoxy)-phenyl, o-, m- oder p-(Fluormethoxy)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2-Chlor-3-methyl-, 2-Chlor-4-methyl-, 2-Chlor-5-methyl-, 2-Chlor-6-methyl-, 2-Methyl-3-chlor-, 2-Methyl-4-chlor-, 2-Methyl-5-chlor-, 2-Methyl-6-chlor-, 3-Chlor-4-methyl-, 3-Chlor-5-methyl- oder 3-Methyl-4-chlorphenyl, 2-Brom-3-methyl-, 2-Brom-4-methyl-, 2-Brom-5-methyl-, 2-Brom-6-methyl-, 2-Methyl-3-brom-, 2-Methyl-4-brom-, 2-Methyl-5-brom-, 2-Methyl-6-brom-, 3-Brom-4-methyl-, 3-Brom-5-methyl- oder 3-Methyl-4-bromphenyl, 2,4- oder 2,5-Dinitrophenyl, 2,5- oder 3,4-Dimethoxyphenyl, 3-Nitro-4-chlorphenyl, 2,3,4-, 2,3,5-, 2,3,6-, 2,4,6- oder 3,4,5-Trichlorphenyl, 2,4,6-tri-tert.-Butylphenyl, ferner bevorzugt 2-Nitro-4-(trifluormethyl)phenyl, 3,5-Di-(trifluormethyl)-phenyl, 2,5-Dimethylphenyl, 2-Hydroxy-3,5-dichlorphenyl, 2-Fluor-5- oder 4-Fluor-3-(trifluormethyl)-phenyl, 4-Chlor-2- oder 4-Chlor-3-(trifluormethyl)-, 2-Chlor-4- oder 2-Chlor 5-(trifluormethyl)-phenyl, 4-Brom-2- oder 4-Brom-3-(trifluormethyl)-phenyl, p-lodphenyl, 2-Nitro-4-methoxyphenyl, 2,5-Dimethoxy-4-nitrophenyl, 2-Methyl-5-nitrophenyl, 2,4-Dimethyl-3-nitrophenyl, 4-Fluor-3-chlorphenyl, 4-Fluor-3,5-dimethylphenyl, 2-Fluor-4-Bromphenyl, 2,5-Difluor-4-bromphenyl, 2,4-Dichlor-5-methylphenyl, 3-Brom-6-methoxyphenyl, 3-Chlor-6-methoxyphenyl, 2-Methoxy-5-methylphenyl oder 2,4,6-Triisopropylphenyl.

R¹ ist auch Het¹.

Het¹ ist vorzugsweise 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 2-, 3-, 4-, 5- oder 6-2H-Thiopyranyl, 2-, 3- oder 4-4-H-Thiopyranyl, 3- oder 4-Pyridazinyl, Pyrazinyl, 2-, 3-, 4-, 5- 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl.

R¹ bedeutet ganz besonders bevorzugt Phenyl, p-Chlorphenyl, p-Fluorphenyl,Thiophen-2-yl, 5-Chlor-thiophen-2-yl, 2,5-Dichlor-thiophen-3-yl und 2- oder 3-Furyl.

R⁴, R⁵ bedeuten jeweils unabhängig voneinander vorzugsweise H, Hal, Alkyl mit 1-6 C-Atomen, Alkoxy mit 1-6 C-Atomen oder Hydroxy, ferner Cyan oder Acyl.

R⁴ bedeutet vorzugsweise H, Hal, A, OA, OH, CN, Acyl, CONH₂ oder CONHA . R⁵ bedeutet vorzugsweise H.

Bevorzugt sind solche Verbindungen der Formel I, in denen der R¹-CH₂-CH₂-piperazin-carbonyl-Rest die 4-, 5-, 6- oder 7-Stellung des Indolrings substituiert.

Dementsprechend ist Gegenstand der Erfindung insbesondere die Verwendung derjenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ih ausgedrückt werden, die der Formel I entsprechen und worin die nicht näher bezeichneten Reste die bei der Formel I angegebene Bedeutung haben, worin jedoch
in Ia
- R¹: Phenyl bedeutet;
in Ib
- R¹: unsubstituiertes oder einfach durch Hal substituiertes Phenyl bedeutet;
in Ii
- R¹: unsubstituiertes oder einfach durch Hal substituiertes Phenyl, Naphtyl oder Het¹,
- R⁴: jeweils unabhängig voneinander H, Hal, CN, Acyl, A oder CONH₂,
- R⁵: H
- R⁴ und R⁵: zusammen auch Alkylen mit 3-5 C-Atomen,
- Het¹: unsubstituiertes oder ein- oder zweifach durch Hal oder A substituiertes Thienyl oder Furyl, bedeutet.

Vorzugsweise werden erfindungsgemäß die folgenden Verbindungen verwendet, die in der WO 01/07435 - gegebenenfalls in der Form eines ihrer Salze - näher charakterisiert sind:
(1 H-Indol-4-yl)-(4-phenethyl-piperazin-1-yl)-methanon,
(1 H-Indol-4-yl)-[4-(4-fluorphenethyl)-piperazin-1-yl]-methanon,
(1 H-Indol-4-yl)-[4-(2,5-dichlor-thiophen-3-ylethyl)-piperazin-1-yl]-methanon,
(3-Formyl-1 H-indol-5-yl)-[4-(4-fluorphenethyl)-piperazin-1-yl]-methanon,
(1H-Indol-6-yl)-[4-(4-fluorphenethyl)-piperazin-1-yl]-methanon,
(1 H-Indol-6-yl)-[4-(thiophen-2-ylethyl)-piperazin-1-yl]-methanon,
Hydrochlorid, F. (1 H-Indol-6-yl)-[4-(2,5-dichlor-thiophen-3-ylethyl)-piperazin-1-yl]-methanon, (3-Cyan-1H-indol-6-yl)-[4-(4-fluorphenethyl)-piperazin-1-yl]-methanon, (1H-Indol-7-yl)-(4-phenethyl-piperazin-1-yl)-methanon,
(1H-Indol-7-yl)-[4-(4-fluorphenethyl)-piperazin-1-yl]-methanon,
(1H-Indol-7-yl)-[4-(5-chlor-thiophen-2-ylethyl)-piperazin-1-yl]-methanon,
(3-Formyl-1H-indol-7-yl)-[4-(4-fluorphenethyl)-piperazin-1-yl]-methanon,
(3-Cyan-1H-indol-7-yl)-[4-(4-fluorphenethyl)-piperazin-1-yl]-methanon,
(2,3-Dimethyl-1H-indol-7-yl)-[4-(4-fluorphenethyl)-piperazin-1-yl]-methanon,
(6,7,8,9-Tetrahydro-5H-carbazol-3-yl)-(4-phenethyl-piperazin-1-yl)-methanon,
(3-Formyl-1H-indol-6-yl)-[4-(4-fluorphenethyl)-piperazin-1-yl]-methanon,
(1H-Indol-6-yl)-[4-(5-chlor-thiophen-2-ylethyl)-piperazin-1-yl]-methanon,
(1H-Indol-4-yl)-[4-(5-chlor-thiophen-2-ylethyl)-piperazin-1-yl]-methanon, (3-Cyan-1H-indol-5-yl)-[4-(4-fluorphenethyl)-piperazin-1-yl]-methanon, (3-Cyan-1H-indol-7-yl)-[4-(naphth-2-ylethyl)-piperazin-1-yl]-methanon, (3-Cyan-1H-indol-4-yl)-[4-(4-fluorphenethyl)-piperazin-1-yl]-methanon, (3-Cyan-1H-indol-4-yl)-[4-(2-fluorphenethyl)-piperazin-1-yl]-methanon, (3-Cyan-1H-indol-7-yl)-[4-(2-fluorphenethyl)-piperazin-1-yl]-methanon, (3-Aminocarbonyl-1H-indol-7-yl)-[4-(4-fluorphenethyl)-piperazin-1-yl]-methanon,
(3-Cyan-1H-indol-7-yl)-[4-(4-fluorphenethyl)-piperazin-1-yl]-methanon,
(3-Cyan-1H-Indol-7-yl)-[4-(5-chlor-thiophen-2-ylethyl)-piperazin-1-yl]-methanon, (3-Cyan-1H-indol-7-yl)-(4-phenethyl-piperazin-1-yl)-methanon,
(3-Cyan-1 H-indol-7-yl)-[4-(2,4-difluorphenethyl)-piperazin-1-yl]-methanon.

Erfindungsgemäß besonders bevorzugt ist die Verwendung von (3-Cyan-1 H-indol-7-yl)-[4-(4-fluorphenethyl)-piperazin-1-yl]-methanon und (3-Aminocarbonyl-1H-indol-7-yl)-[4-(4-fluorphenethyl)-piperazin-1-yl]-methanon.

Ganz besonders bevorzugt ist (3-Cyan-1 H-indol-7-yl)-[4-(4-fluorphenethyl)-piperazin-1-yl]-methanon.

Die Verbindungen der Formel I und Verfahren zu ihrer Herstellung sind aus der WO 01/07435 bekannt. Sie zeigen bei guter Verträglichkeit unter anderem Wirkungen auf das Zentralnervensystem und verfügen dabei über wertvolle pharmakologische Eigenschaften. Die Verbindungen weisen eine starke Affinität zu 5-HT_{2A}-Rezeptoren auf, wobei sie 5-HT_{2A}-Rezeptor-antagonistische Eigenschaften besitzen.

Der Erfindung lag die Aufgabe zugrunde, neue Verwendungen für aus den Verbindungen der Formel I hergestellt Arzneimittel zu finden.

Überraschenderweise wurde gefunden, daß sich die Verbindungen der Formel I zur Behandlung von Fettsucht eignen.

Die Wirksamkeit der Verbindungen der Formel I zur Behandlung von Fettsucht kann *in vivo* wie folgt ermittelt werden (vgl. Beispiel B):

Einer Gruppe von Versuchstieren wird eine bestimmte Dosis oder wechselnde Dosen der Testverbindung über einen längeren Zeitraum verabreicht. Dabei werden die verbrauchte Futtermenge und das Körpergewicht der Versuchstiere regelmäßig protokolliert. Gleichzeitig wird in an sich bekannter Weise das Verhalten und der Allgemeinzustand der Tiere überwacht. Aus der Abnahme des Körpergewichts und der Futteraufnahme im Versuchszeitraum bei ansonsten unverändertem Allgemeinzustand und der Abwesenheit von Verhaltensänderungen können Rückschlüsse auf die Eignung der Testverbindung für die Behandlung der Fettsucht gezogen werden.

Gegenstand der Erfindung ist auch die Verwendung von Verbindungen der Formel I zur Herstellung einer pharmazeutischen Zubereitung zur Behandlung von Fettsucht, enthaltend mindestens ein erfindungsgemäßes Arzneimittel sowie gegebenenfalls Träger- und/oder Hilfsstoffe und gegebenenfalls andere Wirkstoffe.

Hierbei können die Arzneimittel zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Zur Behandlung der Fettsucht werden die erfindungsgemäßen Substanzen in der Regel in Analogie zu bekannten Präparaten verabreicht, vorzugsweise in Dosierungen zwischen etwa 0,1 und 500 mg, insbesondere zwischen 5 und 300 mg pro Dosierungseinheit. Die tägliche Dosierung liegt vorzugsweise zwischen etwa 0,01 und 250 mg/kg, insbesondere zwischen 0,02 und 100 mg/kg Körpergewicht.

Vorzugsweise werden die erfindungsgemäßen Substanzen zur Behandlung von Fettsucht in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Die erfindungsgemäßen Verbindungen können auch zusammen mit anderen Wirkstoffen, in der Behandlung der erwähnten Krankheiten eingesetzt werden.

Eine konkrete Anleitung zur Synthese von Verbindungen der Formel I kann der WO 01/07435 entnommen werden.

Die pharmazeutischen Zubereitungen können als Arzneimittel in der Human- und Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositoren, zur parenteralen Applikation Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenden Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

### Die nachfolgenden Beispiele betreffen pharmazeutische Zubereitungen:

### Beispiel A1: Injektionsgläser

Eine Lösung von 100 g eines Wirkstoffes der Formel I und 5 g Dinatriumhydrogenphosphat in 3 I zweifach destilliertem Wasser wird mit 2 n Salzsäure auf pH 6,5 eingestellt, steril filtriert, in Injektionsgläser abgefüllt, lyophilisiert und steril verschlossen. Jedes Injektionsglas enthält 5 mg Wirkstoff.

### Beispiel A2: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel A3: Lösung

Man bereitet eine Lösung aus 1 g eines Wirkstoffes der Formel I, 9.38 g NaH₂PO₄ x 2 H₂O, 28.48 g NaH₂PO₄ x 12 H₂O und 0.1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel A4: Salbe

Man mischt 500 mg eines Wirkstoffes der Formel I mit 99,5 g Vaseline unter aseptischen Bedingungen.

### Beispiel A5: Tabletten

Ein Gemisch von 1 kg Wirkstoff der Formel I, 4 kg Lactose, 1.2 kg Kartoffelstärke, 0.2 kg Talk und 0.1 kg Magnesiumstearat wird in üblicher Weise zu Tabletten verpreßt, derart, daß jede Tablette 10 mg Wirkstoff enthält.

### Beispiel A6: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel A7: Kapseln

2 kg Wirkstoff der Formel I werden in üblicher Weise in Hartgelatinekapseln gefüllt, so daß jede Kapsel 20 mg des Wirkstoffs enthält.

### Beispiel A8: Ampullen

Eine Lösung von 1 kg Wirkstoff der Formel I in 60 I zweifach destilliertem Wasser wird in Ampullen abgefüllt, unter aseptischen Bedingungen lyophilisiert und steril verschlossen. Jede Ampulle enthält 10 mg Wirkstoff.

### Beispiel B: Behandlung von Hunden mit (3-Cyan-1H-indol-7-yl)-[4-(4-fluorphenethyl)-piperazin-1-yl]-methanon, Hydrochlorid

Über einen Zeitraum von 11 Tagen wird die Testsubstanz einer Gruppe von etwa 8 Monate alten, zuvor entwurmten Beagle-Hunden einmal täglich in Form von gemäß Beispiel B7 hergestellten Hartgelatinekapseln verabreicht. Die aufgenommene Futtermenge wird täglich durch Rückwiegen des unverbrauchten Futters, das Körpergewicht vor (Tag 0), während (Tag 5) und nach (Tag 12) des Fütterungsversuchs ermittelt. Gleichzeitig wird über den gesamten Versuchszeitraum das Verhalten und der Allgemeinzustand der Tiere überwacht. Trinkwasser steht den Hunden in unbegrenzter Menge zur Verfügung.

Wie aus den in Tabelle 1 zusammengefaßten Versuchsergebnissen ersichtlich, schränken die Hunde im Verlauf des Versuchs die Futteraufnahme zunehmend ein. Dies geht mit einer deutlichen Reduktion des Körpergewichts einher. Verhalten und Allgemeinzustand der Hunde werden durch die Verabreichung der Testsubstanz im Versuchszeitraum nicht beeinträchtigt.

**Tabelle 1: Fütterungsversuch an Beagle-Hunden mit (3-Cyan-1H-indol-7-yl)-[4-(4-fluorphenethyl)-piperazin-1-yl]-methanon, Hydrochlorid (¹: Körpergewicht in kg, ²: Futterverbrauch in g).**

| Tag | 0 | 1 | 2 | 3 | 4 | 5 | 6 | 7 | 8 | 9 | 10 | 11 | 12 |
|---|---|---|---|---|---|---|---|---|---|---|---|---|---|
| | | | | | | | | | | | | | |
| Dosis mg/kg | | 3 | 10 | 30 | 100 | 100 | 100 | 100 | 100 | 100 | 100 | 100 | |
| Hund 8910 (männlich)¹ | 8.6 | | | | | 8.6 | | | | | | | 7.8 |
| Hund 8936 (weiblich)¹ | 7.5 | | | | | 7.3 | | | | | | | 6.2 |
| Dose mg/kg | | | | | | 50 | 50 | 50 | 50 | 50 | 50 | 50 | |
| Hund 1201 (männlich)¹ | 6.8 | | | | | 7.1 | | | | | | | 6.3 |
| Hund 2164 (weiblich)¹ | 6.1 | | | | | 5.9 | | | | | | | 5.5 |
| | | | | | | | | | | | | | |
| Dosis mg/kg | | 3 | 10 | 30 | 100 | 100 | 100 | 100 | 100 | 100 | 100 | 100 | |
| Hund 8910 (männlich)² | | 380 | 320 | 370 | 320 | 250 | 240 | 60 | 60 | 40 | 20 | 90 | |
| Hund 8936 (weiblich)² | | 380 | 380 | 360 | 220 | 200 | 140 | 80 | 30 | 30 | 70 | 10 | |
| Dosis mg/kg | | | | | | 50 | 50 | 50 | 50 | 50 | 50 | 50 | |
| Hund 1201 (männlich)² | | 440 | 260 | 400 | 230 | 340 | 220 | 160 | 170 | 130 | 80 | 40 | |
| Hund 2164 (weiblich)² | | 310 | 270 | 280 | 280 | 340 | 180 | 260 | 150 | 140 | 130 | 60 | |

## Patentansprüche

1. Verwendung von Verbindungen der Formel I 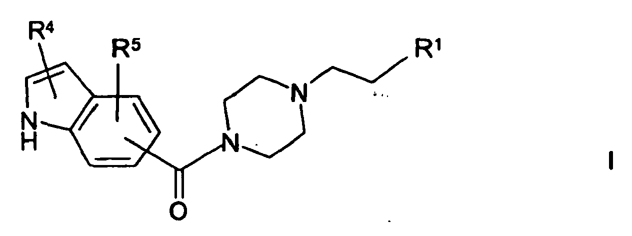 worin
R¹ einen unsubstituierten oder durch R² und/oder R³ substituierten Phenylrest oder Naphtylrest
oder Het¹,
_{R}², R³ jeweils unabhängig voneinander Hal, A, OA, OH oder CN,
R⁴, R⁵ jeweils unabhängig voneinander H, CN, Acyl, Hal, A, OA, OH, CONH₂, CONHA oder CONA₂,
R⁴ und R⁵ zusammen auch Alkylen mit 3-5 C-Atomen,
Het¹ ein- oder zweikerniges unsubstituiertes oder ein- oder zweifach durch Hal, A, OA oder OH substituiertes ungesättigtes heterocyclisches Ringsystem, welches eines, zwei oder drei gleiche oder verschiedene Heteroatome wie Stickstoff, Sauerstoff und Schwefel enthält,
A Alkyl mit 1-6 C-Atomen,
Hal F, Cl, Br oder I,
bedeuten,
sowie deren physiologisch unbedenklichen Salzen und Solvaten zur Herstellung eines Arzneimittels zur Behandlung von Fettsucht.

2. Verwendung von Verbindungen der Teilformel Ib der Formel I gemäß Anspruch 1, worin R¹ unsubstituiertes oder einfach durch Hal substituiertes
Phenyl bedeutet
und die Reste R⁴, R⁵ und Hal die in Anspruch 1 angegebene Bedeutung haben, sowie deren physiologisch unbedenklichen Salzen und Solvaten zur Herstellung eines Arzneimittels zur Behandlung von Fettsucht.

3. Verwendung von Verbindungen der Teilformel Ii der Formel I gemäß Anspruch 1, worin
R¹ unsubstituiertes oder einfach durch Hal substituiertes Phenyl, Naphtyl oder Het¹,
R⁴ jeweils unabhängig voneinander H, Hal, CN, Acyl, A oder CONH₂,
R⁵ H,
R⁴ und R⁵ zusammen auch Alkylen mit 3-5 C-Atomen,
Het¹ unsubstituiertes oder ein- oder zweifach durch Hal oder A substituiertes Thienyl oder Furyl
bedeutet
sowie deren physiologisch unbedenklichen Salzen und Solvaten zur Herstellung eines Arzneimittels zur Behandlung von Fettsucht.

4. Verwendung von Verbindungen der Formel I gemäß Anspruch 1, ausgewählt aus
(a) (3-Cyan-1H-indol-7-yl)-[4-(4-fluorphenethyl)-piperazin-1-yl]-methanon,
(b) (3-Aminocarbonyl-1H-indol-7-yl)-[4-(4-fluorphenethyl)-piperazin-1-yl]-methanon
sowie deren physiologisch unbedenklichen Salzen und Solvaten
zur Herstellung eines Arzneimittels zur Behandlung von Fettsucht.

## Claims

1. Use of compounds of the formula I 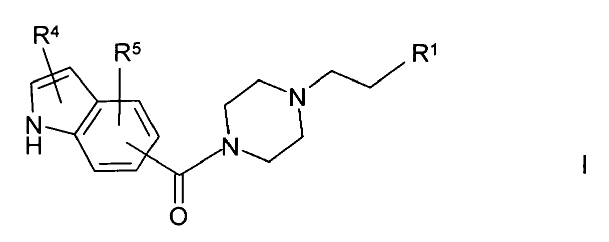 in which
R¹ denotes a phenyl radical or naphthyl radical, each of which is unsubstituted or substituted by R² and/or R³,
or Het¹,
R², R³ each, independently of one another, denote Hal, A, OA, OH or CN,
R⁴, R⁵ each, independently of one another, denote H, CN, acyl, Hal, A, OA, OH, CONH₂, CONHA or CONA₂,
R⁴ and R⁵ together also denote alkylene having 3-5 C atoms,
Het¹ denotes a mono- or bicyclic, unsaturated heterocyclic ring system which is unsubstituted or mono- or disubstituted by Hal, A, OA or OH and which contains one, two or three identical or different heteroatoms, such as nitrogen, oxygen and sulfur,
A denotes alkyl having 1-6 C atoms,
Hal denotes F, CI, Br or I,
and physiologically acceptable salts and solvates thereof, for the preparation of a medicament for the treatment of obesity.

2. Use of compounds of the sub-formula Ib of the formula I according to Claim 1, in which R¹ denotes phenyl which is unsubstituted or monosubstituted by Hal,
and the radicals R⁴, R⁵ and Hal have the meaning indicated in Claim 1, and physiologically acceptable salts and solvates thereof, for the preparation of a medicament for the treatment of obesity.

3. Use of compounds of the sub-formula li of the formula I according to Claim 1, in which
R¹ denotes phenyl, naphthyl, each of which is unsubstituted or monosubstituted by Hal, or Het¹,
R⁴ in each case, independently of one another, denotes H, Hal, CN, acyl, A or CONH₂,
R⁵ denotes H,
R⁴ and R⁵ together also denote alkylene having 3-5 C atoms,
Het¹ denotes thienyl or furyl, each of which is unsubstituted or mono- or disubstituted by Hal or A,
and physiologically acceptable salts and solvates thereof, for the preparation of a medicament for the treatment of obesity.

4. Use of compounds of the formula I according to Claim 1, selected from
(a) (3-cyano-1H-indol-7-yl)-[4-(4-fluorophenethyl)piperazin-1-yl]-methanone,
(b) (3-aminocarbonyl-1H-indol-7-yl)-[4-(4-fluorophenethyl)piperazin-1-yl]methanone
and physiologically acceptable salts and solvates thereof, for the preparation of a medicament for the treatment of obesity.

## Revendications

1. Utilisation de composés de formule I 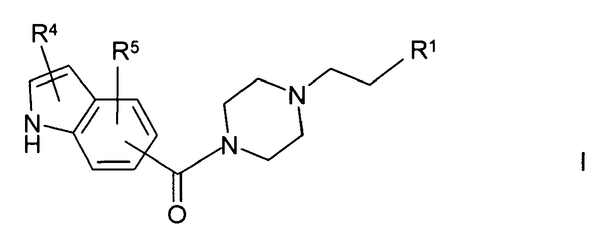 dans laquelle
R¹ désigne un radical phényle ou un radical naphtyle, chacun d'entre eux étant non substitué ou substitué par R² et/ou R³,
ou Hét¹,
R², R³ désignent chacun, indépendamment l'un de l'autre, Hal, A, OA, OH ou CN,
R⁴, R⁵ désignent chacun, indépendamment l'un de l'autre, H, CN, acyle, Hal, A, OA, OH, CONH₂, CONHA ou CONA₂,
R⁴ et R⁵ désignent également ensemble alkylène ayant 3-5 atomes de C,
Hét¹ désigne un noyau hétérocyclique insaturé mono- ou bicyclique, qui est non substitué ou mono- ou disubstitué par Hal, A, OA ou OH et qui contient un, deux ou trois hétéroatomes identiques ou différents, tels que l'azote, l'oxygène et le soufre,
A désigne alkyle ayant 1-6 atomes de C,
Hal désigne F, CI, Br ou I,
et les sels et solvats physiologiquement acceptables de ceux-ci, pour la préparation d'un médicament destiné au traitement de l'obésité.

2. Utilisation de composés de sous-formule Ib de la formule I selon la revendication 1, dans laquelle R¹ désigne phényle qui est non substitué ou monosubstitué par Hal,
et les radicaux R⁴, R⁵ et Hal ont la signification indiquée selon la revendication 1, et les sels et solvats physiologiquement acceptables de ceux-ci, pour la préparation d'un médicament destiné au traitement de l'obésité.

3. Utilisation de composés de sous-formule li de la formule I selon la revendication 1, dans laquelle
R¹ désigne phényle, naphtyle, chacun d'entre eux étant non substitué ou monosubstitué par Hal, ou Hét¹,
R⁴ dans chaque cas, indépendamment l'un de l'autre, désigne H, Hal, CN, acyle, A ou CONH₂,
R⁵ désigne H,
R⁴ et R⁵ désignent également ensemble alkylène ayant 3-5 atomes de C,
Hét¹ désigne thiényle ou furyle, chacun d'entre eux étant non substitué ou mono- ou disubstitué par Hal ou A,
et les sels et solvats physiologiquement acceptables de ceux-ci, pour la préparation d'un médicament destiné au traitement de l'obésité.

4. Utilisation de composés de formule I selon la revendication 1, choisis parmi
(a) la (3-cyano-1H-indol-7-yl)-[4-(4-fluorophénéthyl)pipérazin-1-yl]-méthanone,
(b) la (3-aminocarbonyl-1H-indol-7-yl)-[4-(4-fluorophénéthyl)pipérazin-1-yl]méthanone
et les sels et solvats physiologiquement acceptables de ceux-ci, pour la préparation d'un médicament destiné au traitement de l'obésité.
